# EUROPEAN PATENT SPECIFICATION

(11) **EP 1 077 674 B1**
(45) Date of publication and mention of the grant of the patent: **06.12.2006**
(21) Application number: 99925035.0
(22) Date of filing: 21.05.1999
(51) Int. Cl.: A61K 8/27, A61Q 19/00, A61Q 15/00, A61K 33/30

(54) **USE OF A TOPICAL COMPOSITION**
VERWENDUNG EINER TOPISCHEN ZUSAMMENSETZUNG
UTILISATION D'UNE COMPOSITION TOPIQUE

(30) Priority: 21.05.1998 GB 9810803
(43) Date of publication of application: 28.02.2001
(73) Proprietor: The Boots Company PLC, Nottingham, Nottinghamshire NG2 3AA (GB)
(72) Inventor: BUTCHER, Kate Elizabeth, Nottingham NG2 3AA (GB); DE GRAAF, Thalie Paulina, Nottingham NG2 3AA (GB); GALLEY, Edward, Nottingham NG2 3AA (GB)
(74) Representative: Jones, Stephen Anthony
(86) International application number: PCT/EP1999/003590
(87) International publication number: WO 1999/059538

(56) References cited:
- EP-A- 0 564 307
- WO-A-95/13806
- GB-A- 2 233 227
- US-A- 4 239 781
- PATENT ABSTRACTS OF JAPAN vol. 1996, no. - , 26 December 1996 (1996-12-26) & JP 08 217637 A (KAO CORP), 27 August 1996 (1996-08-27)
- PATENT ABSTRACTS OF JAPAN vol. 1999, no. 053, 31 May 1999 (1999-05-31) & JP 11 049637 A (KAO CORP), 23 February 1999 (1999-02-23)
- PATENT ABSTRACTS OF JAPAN vol. 1996, no. - , 29 February 1996 (1996-02-29) & JP 07 277914 A (KAO CORP), 24 October 1995 (1995-10-24)
- DATABASE WPI Week 8402 Derwent Publications Ltd., London, GB; AN 1984-007489 XP002116894 & JP 48 056818 A (OIKAWA T.), 9 August 1973 (1973-08-09)

## Description

This invention teaches the use of a new form of zinc oxide as an agent to absorb liquids on human skin.

The skin is one of the most complex organs in the human body. As part of a myriad of processes, it exudes sweat and sebum. An excess of sweat or sebum is implicated in a number of unpleasant human conditions such as acne, greasy hair/skin and body odour, unless it is removed by washing or some other means.

Further a lack of care for the skin, in particular not keeping it dry may result in a number of irritating conditions. Moisture between the toes such as water or sweat provides a fertile breeding ground for the microbes that cause athletes foot. Similarly, the prolonged dampness that results from not changing a baby's nappy frequently or not drying its buttocks sufficiently often gives rise to nappy rash.

There are a number of approaches to combating the problems of liquids on the skin.

Where the liquid is sweat, a common approach is to block the sweat pores with antiperspirants. The problem of odour associated with an excess of sweat or sebum being broken down by the micro-organisms present on the skin can be overcome with odour masking agents such as deodorants.

Another approach is to use an agent to absorb the liquid. Talcs, clays and silica have all been used to absorb sebum. Talcs are also used to absorb sweat in foot products, and to keep babies bottoms dry.

This invention describes the use of a new form of zinc oxide in topical compositions which has vastly superior absorbence properties. This form of zinc oxide has a surface area between 30m²/g and 100m²/g, preferably greater than 90m²/g. The particles have an average diameter between 0.1 and 200^{µ}m, preferably between 0.1 and 20.5^{µ}m. Hereinafter, such zinc oxide particles shall be known as "high surface area zinc oxide". The surface area of the material can be determined using laser diffraction. From the surface area, the mean estimated spherical diameter may be calculated. The diameter of the particles is henceforth taken to mean the mean estimated spheric diameter. The surface area of the high surface area zinc oxide particles used with this invention was calculated using a Micromeritics Flavsorb II 2300 BET apparatus. Such zinc oxide is commercially available from Elementis as Activox C80.

The production of zinc oxides suitable for use in the topical compositions of the present invention is well documented.

### WO 95/04704 (Harcros)

This patent discloses a method for the production of zinc oxide in the form of discrete particles which have an average particle size of 0.08 µm or less in diameter and a surface area >12.5m²/g. The zinc oxide produced is found to be particularly good for use as an additive in skin formulations designed for scattering/absorbing UV light.

### S. Tichy, SOFW - Journal 119 Jahrgang 8/93

This article teaches a method for producing zinc oxide with a particle size of 200 µm and a surface area of 50-150m²/g.

### Liu et al - Journal of Materials Science 21 (1986) 3698-3702

This describes another method of producing zinc oxide particles with a diameter of 0.15 µm and a surface area of about 50m²/g.

These articles are but a small selection from a large number of articles detailing the manufacture of these modified zinc oxide particles.

EP-A-603627 discloses a process for preparing microfine zinc oxide particle diameter is 5 to 100 nm and BET-surface area of from 30 to 100 m²/g for use as a sunscreen.

Patent Abstracts of Japan of JP-A-08 217637 discloses a powder cosmetic in which a free powder (which may be ZnO) of granular diameter 0.1-2 µm is located on the surface of spherical powder (which may be ZnO) of granular diameter 3-50 µm and may have a surface area of 15-100 µ²/g.

GB-A-2233227 discloses the use of zinc oxide in a topical aqueous solution to treat foot odour. No disclosure of surface area or particle size. It appears that formulations according to the British Phamacopaeia were intended.

EP-A-564307 discloses the use of buffered formulations to prevent skin rash. The formulations may contain zinc oxide as a pH stabiliser or a urease suppressor or inhibitor but no surface area or particle size is specified.

WO-A-95/13806 discloses compositions to treat skin disorders containing zinc linoleate and linoleic acid. Zinc oxide is used as a starting material but in the preparation of the composition essentially all of the zinc oxide is converted into the linoleate so no zinc oxide remains in the compositions.

Patent Abstracts of Japan of JP-A-07 277914 discloses cosmetic composition containing (a) a fluorine-modified silicone, (b) a zinc oxide having a surface area of 10-100 m²/g and (c) a powder treated with a fluorine compound. The composition is said to prevent cosmetic ingredients from disappearing due to perspiration or sebum'. No particle size is given for the zinc oxide.

US-A-4239781 discloses treating skin ailments by applying lower polyalkylene glycols. Zinc oxide may be present solely as a filler.

Derwent Abstract 1984-007489 of JP-A-48056818 discloses preparation or treating athletes foot containing ZnO. No surface area or particle size is given.

This invention teaches the use of the high surface area zinc oxide particles to absorb sebum from human skin.

Surprisingly, high surface area zinc oxide has been found to be highly efficacious at absorbing straight chain fatty acids found in sebum from the skin. Excess sebum upon the skin is implicated as a factor in a number of unpleasant human conditions. These include acne, greasy skin, greasy hair, sweaty feet, athletes foot, body odour and nappy rash.

High surface area zinc oxide's high absorbency and gentleness upon the skin make it particularly suitable for use in cosmetic compositions. Cosmetic compositions containing high surface area zinc oxide applied to a part of the body would absorb the liquid present on the skin. Thus, the cosmetic compositions would be capable of acting to treat or prevent many of the socially unacceptable problems which are prevalent in those with an excess of liquid upon their skin.

The present invention provides the use of high surface area zinc oxide as defined in Claim 1.

Preferably, the surface area of the high surface area zinc oxide is greater than 90m²/g and the average particle size is between 0.1 and 20.5µm in diameter.

In preferred topical formulations, the high surface area zinc oxide is present from 3 to 8%, most preferably 4 to 6% by weight of the total composition.

Acne is a common affliction of many people in their teenage years and sometimes beyond. As a result of puberty, teenagers often have increased levels of sebum. The initial inflammation of the follicle wall in the development of acne results from the presence of free fatty acids derived from the sebum. The normal bacterial flora in the sebaceous duct produce the enzymes responsible for splitting triglycerides in the sebum and releasing these fatty acids. The main micro-organisms in the sebaceous duct are *Propionibacterium acnes* and one or two species of *Staphyloccus aureus.* Therefore in the presence of excess sebum these micro-organisms may result in the development of acne.

Most approaches to a cure for acne focus on trying to absorb the excess sebum or to act on the bacteria present.

High surface area zinc oxide particles have been found to be particularly efficacious in the absorption of the straight chain fatty acids found in sebum particularly those of longer chain length. It is these compounds which are particularly implicated in many of the causes of acne. By absorbing these compounds from the skin, high surface area zinc oxide can prevent the formation of pustules and comedones. This invention teaches the use of high surface area zinc oxide in the treatment of acne. High surface area zinc oxide may be used in the preparation of a medicament for the treatment of acne.

Athletes foot is the loose term applied to a skin eruption on the foot, usually between the toes. It is a cutaneous fungal infection, most commonly caused by *Tricophyton rubrum, Tricophyton mentagrophytes* or *Epidermophyton floccosum.* In addition to the effect of the micro-organisms, other factors such as wetness or an increase in temperature can contribute to disease development by providing ideal conditions for the initiation and growth of fungal infections. The condition is commonly treated by careful foot hygiene, removing the damp conditions helpful to fungal growth and by the use of antifungal agents. High surface area zinc oxide has been found to be highly absorbent of liquids. By absorbing sweat or liquid, particularly sweat from the skin of the foot, this invention teaches the use of high surface area zinc oxide to treat athletes foot. High surface area zinc oxide may be used in the preparation of a medicament for the treatment of athletes foot.

Nappy rash, the skin eruption which tends to occur on the buttocks of infants is due to infrequent changing of soiled nappies. The condition is often worsened by secondary infection with *Candida albicans.* To prevent this condition, nappies are changed regularly and care is taken to ensure that the baby's bottom is dried properly. The buttocks are commonly treated with an agent to absorb any surplus liquid. High surface area zinc oxide has been found to be highly efficacious in absorbing liquid on the skin in this region. This invention teaches the use of high surface area zinc oxide to prevent and treat nappy rash. High surface area zinc oxide may be used in the preparation of a medicament for the treatment of nappy rash.

In an embodiment of the invention, the cosmetic formulation may be suitable for application as an deodorant. The deodorant may be in the form of a roll on, a spray or other suitable form. Such compositions may be formulated in a manner known to those skilled in the art, and may include, though not limited to:-
a) antiperspirants such as aluminium zirconium, aluminium chlorohydrate, aluminium zirconium pentachlorohydrate.
b) emulsifiers such as steareth-2, steareth-21 and polypropylene glycol-15 stearyl ether.
c) thickeners such as cyclomethicone, dimethicone copolyol, hydroxypropyl methylcellulose, hydroxypropylcellulose.
d) humectants such as propylene glycol, butylene glycol and glycerin.

In a further embodiment of the invention, the cosmetic formulation may be a shampoo. The composition may be formulated in a manner known to those skilled in the art. Such compositions may include, though not limited to:-
a) surfactants such as cocamidopropyl betaine and sodium laureth sulphate.
b) thickeners such as xanthan gum or hydroxyethylcellulose, laureth-3, polyethylene glycol-40 hydrogenated castor oil, polyethylene glycol-55 propylene glycol oleate and propylene glycol.
c) pearl concentrates such as formaldehyde, a blend of stearic acid, cocamide MEA, glycol distearate and glycol stearate, methyldibromo glutaronitrile.
d) conditioners such as polyquaternium-39 and polyquaternium-7, hydroxypropyl guar hydroxypropyltrimonium chloride, polyquaternium-10.
e) preservatives such as paraben, phenoxyethanol with methyldibromo glutaronitrile.
f) perfumes.
g) colour.

In another embodiment of the invention, the cosmetic formulation may be a gel. The composition may be formulated in a manner known to those skilled in the art. Such compositions may include, though not limited to:-
a) antibacterials such as dichlorobenzyl alcohol, triclosan, chlorhexidine digluconate and salicylic acid.
b) alcohols such as denatured ethanol and isopropyl alcohol.
c) humectants such as panthenol, butylene glycol, glycerin and propylene glycol.
d) preservatives such as methyldibromo glutaronitrite, phenoxyethanol, magnesium chloride, magnesium nitrate, methylchloroisothiazoline, any paraben and methylisothiazolinone.
e) solubilisers such as polysorbate 20, polypropylene glycol-40 hydrogenated castor oil.
f) cooling agents such as Hamamelis virginiana solution and Mentha piperita.
h) emollients such as glycerin, propylene glycol and butylene glycol.
i) oil absorbers such as silica.
i) thickeners such as xanthan gum, hydroxyethylcellulose, sodium magnesium silicate.

In another embodiment of the invention, the cosmetic formulation may be a powder, such as, but not limited to, foot powder or face powder. The composition may be formulated in a manner known to those skilled in the art. Such compositions may include, though not limited to:-
a) preservative such as any paraben, such as methyl paraben, ethyl paraben, propyl paraben and butyl paraben.
b) anti-caking agent/lubricants such as magnesium stearate, calcium stearate and stearic acid.
c) binders such as paraffinum liquidum and any waxes and oils.
d) powders such as sanitised talc.
f) colours
g) absorbents such as Zea mays, rice starch and sodium bicarbonate.
h) flow agents such as silica.

In a further embodiment of the invention, the cosmetic formulation may be a skin wash, such as a cleanser, moisturiser, face wash, lotion, stick or cream. The composition may be formulated in a manner known to those skilled in the art. Such compositions may include, though not limited to:-
a) alcohols such as isopropyl myristate, stearyl alcohol, denatured ethanol.
b) emulsifiers such as steareth-2, glyceryl stearate, hydrogenated vegetable glycerides, steareth-21, ceteth-20, cetyl alcohol, cetearyl alcohol, stearic acid, paraffin, stearyl alcohol, polawax, tribehenin, ceteareth-7, ceteth-5.
c) emollients such as polypropylene glycol-5-ceteth-20, methyl gluceth-10, dicaprylyl maleate, cetearyl isononanoate, silicones, paraffinum liquidum, octyl palmitate, petrolatum, dioctyl maleate, isohexadecane, cetearyl octanoate and isopropyl myristate.
d) solubilisers such as polysorbate 80, polysorbate 20, polyethylene glycol-40 hydrogenated castor oil, any polysorbate.
e) antibacterials such as triclosan, chlorhexidine digluconate, salicylic acid, dichlorobenzyl alcohol.
g) thickeners such as hydroxyethylcellulose, xanthan gum, sodium magnesium silicate, magnesium aluminium silicate, cellulose.
i) detergents such as sodium laureth sulfate, ammonium lauryl sulfate, magnesium lauryl sulfate, disodium undecylenamido MEA-sulfosuccinate.
j) preservatives such as phenoxyethanol, 2-bromo-2-nitropropane-1,3-diol, methyldibromo glutaronitrile, imidazolidinyl urea, magnesium chloride, magnesium nitrate, methylchloroisothiazolinone, methylisothiazolinone or any paraben, such as butyl paraben, ethyl paraben, methyl paraben and propyl paraben.
j) absorbents such as hydrated silica, clays, talcs.
k) antioxidants such as butylated hydroxytoluene or butylated hydroxyacetone.
I) moisturisers such as butylene glycol, propylene glycol, sorbitol and glycerin, panthenol, sodium hyaluronate, sodium PCA.

The efficacy of high surface area zinc oxide as an absorbent has been demonstrated by *in vivo* and *in vitro* trials. The suitability of high surface area zinc oxide for the purposes stated herein has been demonstrated by trials of standard control formulations against those formulations containing high surface area zinc oxide.

The invention is further illustrated by way of the following non-limiting examples:-
Unless otherwise stated, the zinc oxide used in the following examples has an average surface area of 90m²/g and an average particle diameter of 10.47^{µ}m.

### Example 1 - Foot Powder + High Surface Area Zinc Oxide

| Ingredient | % |
|---|---|
| Sanitised talc | 47.46 |
| High Surface Area Zinc Oxide | 5.00 |
| Zea Mays | 47.46 |
| Silica | 0.08 |

Silica and half the sanitised talc were sifted through a 40 mesh sieve into a bowl containing the high surface area zinc oxide, then mixed until thoroughly dispersed.

The remaining sanitised talc was sifted into a Simon Solitec Mixer via a 20 mesh sieve. To this was added the high surface area zinc oxide mixture, via a 40 mesh sieve. The Zea Mays was added and the mixture stirred for 10 minutes. A fifth of the mixture was then removed and sifted back into the mixer via a 20 mesh sieve. The mixture was then stirred for a further 20 minutes.

### Example 2 - Foot Powder Control

| Ingredient | % |
|---|---|
| Sanitised talc | 50.00 |
| Zea Mays | 50.00 |

Ingredients were sieved through a 40 mesh sieve and then mixed for 20 minutes.

### Example 3 - Foot Powder with High Surface Area Zinc Oxide

| Ingredient | % |
|---|---|
| Sanitised Talc | 47.5 |
| Zea Mays | 47.5 |
| High surface area zinc oxide | 5.0 |

Ingredients were sieved through a 40 mesh sieve and then mixed for 20 minutes.

### Example 4 - Roll-on Deodorant + High Surface Area Zinc Oxide

| Ingredients | % |
|---|---|
| High Surface Area Zinc Oxide | 5.00 |
| Polypropylene glycol-15 stearyl ether | 4.00 |
| Steareth-21 | 1.50 |
| Steareth-2 | 2.20 |
| Aluminium Chlorohydrate Solution | 30.00 |
| Tetrasodium EDTA (Sequestrene) | 0.10 |
| Perfume | 0.50 |
| Colour | qs |
| Purified Water | to 100 |

Tetrasodium EDTA was added to purified water, mixed with a homogeniser for 1 minute, then warmed to 70-75°C, until all the solids had dissolved.

Polypropylene glycol - 15 stearyl ether, steareth-21, steareth-2 and high surface area zinc oxide were mixed and heated to 70-75°C prior to addition of tetrasodium EDTA in water. The mixture was homogenised for 5 minutes then stirred slowly whilst cooling to 35°C. Then the aluminium chlorohydrate solution was slowly added to the stirred solution, followed by the perfume. The mixture was stirred until homogeneous, then the colour solution was added.

### Example 5 - Oily Scalp Shampoo + High Surface Area Zinc Oxide

| Ingredient | % |
|---|---|
| Panthenol | 0.20 |
| Xanthan Gum | 0.40 |
| Sodium Laureth Sulfate | 35.00 |
| Cocamidopropyl betaine 30 % | 1.50 |
| Citric acid | 0.08 |
| Polyquaternium - 39 | 0.50 |
| Laureth - 3 | 1.00 |
| Silk protein complex | 0.05 |
| Preservative | 0.20 |
| Perfume | 0.50 |
| Colour | qs |
| Salt | 1.62 |
| Pearling agent blend consisting of: Formaldehyde, glycol distearate, laureth - 10, cocamide MEA and sodium laureth sulfate | 4.00 |
| Purified Water | 52.95 |
| High Surface Area Zinc Oxide | 2 |

High surface area zinc oxide was added to a solution of citric acid in water, and mixed for 15 minutes. Xanthan gum was then added and the mixture homogenised for 15 minutes. To the mixture was added sodium laureth sulfate, cocamidopropyl betaine 30%, laureth-3, panthenol, polyquaternium-39, silk protein complex, perfume and preservative and the pearling agent blend whilst stirring. Colour solution was added and then cold water to make up to bulk. The mixture was then stirred until uniform.

### Example 6 - Mens Facial Wash + High Surface Area Zinc Oxide

| Ingredient | % |
|---|---|
| High Surface Area Zinc Oxide | 5.00 |
| Hydroxyethylcellulose | 1.25 |
| Sodium laureth sulfate | 6.57 |
| Disodium undecylenamido sullfosuccinate | 1.00 |
| Butylene Glycol | 2.00 |
| Preservative | 0.80 |
| Benzoic Acid | 0.10 |
| Polysorbate 20 | 2.00 |
| Perfume | 0.40 |
| Herbal extract | 0.60 |
| Colours | qs |
| Purified Water | to 100 |

### Stage 1

In a stainless steel vessel butylene glycol and preservative were mixed together until uniform.

### Stage 2

In another container the perfume and polysorbate 20 and high surface area zinc oxide were mixed together until uniform.

### Stage 3

To a stainless steel container was added some of the water and hydroxyethylcellulose. The mixture was stirred for 20-30 minutes until fully dispersed.

Stage 3 was then added to stage 1, followed by the herbal extract, benzoic acid and colour. The mixture was stirred until fully dispersed, then sodium laureth sulfate, and sodium undecylenamido MEA-sulfosuccinate were added to the stirred mixture. Stage 2 was added and mixed thoroughly. Cold water was added to make up to bulk.

### Example 7 - Skin Treatment Gel + High Surface Area Zinc Oxide

| Ingredients | % |
|---|---|
| Alcohol denatured | 10.00 |
| Allantoin | 0.10 |
| Glycerin | 1.00 |
| Butylene Glycol | 4.00 |
| Xanthan Gum | 1.00 |
| Phenoxyethanol | 0.20 |
| Hydrated silica | 0.50 |
| Dichlorobenzyl alcohol | 0.10 |
| Colour | qs |
| Benzophenone - 4 | 0.10 |
| Purified Water | to 100 |
| Panthenol | 0.50 |
| High Surface Area Zinc Oxide | 5.00 |

Xanthan gum, dispersed in 2% of the butylene glycol was added to some of the purified water, and mixed together for 30 minutes. To this mixture allantoin, sequestrene and panthenol were added and the mixture stirred for 5 minutes. The mixture was cooled to 35°C, then premixed phenoxyethanol and glycerin were added to the mixture, followed by premixed alcohol (denatured) and purified water followed by premixed dichlorobenzyl alcohol and butylene glycol. The mixture was then stirred. Benzophenone-4 and water was then added with stirring, followed by hydrated silica and high surface area zinc oxide. The mixture was stirred, cooled to below 35°C and then the colour was added. Cold water was added to make up to bulk, and the mixture stirred for a further 30 minutes.

### Example 8 - Non-oily Moisturiser + High Surface Area Zinc Oxide

| Ingredients | % |
|---|---|
| High Surface Area Zinc Oxide | 5.00 |
| Colour | qs |
| Perfume | 0.10 |
| Triclosan | 0.10 |
| Allantoin | 0.10 |
| Phenoxyethanol | 0.20 |
| Hydroxyethylcellulose | 2.00 |
| Polysorbate 20 | 1.00 |
| Butylene Glycol | 3.50 |
| Glycerin | 4.50 |
| Purified Water | to 100 |

### Stage 1

To purified water in a homogeniser hydroxyethylcellulose was added and then homogenised for at least 30 minutes. The homogeniser was switched off and with stirring allantoin and phenoxyethanol, which had been previously dissolved in glycerin and butylene glycol, were added.

### Stage 2

Butylene glycol and glycerin were warmed together to 45°C. Then with stirring triclosan and high surface area zinc oxide were added and completely dissolved and cooled to 35°C.

### Stage 3

Using a homogeniser stage 2 was added to stage 1 and homogenised for 10 minutes. The perfume, previously dispersed in polysorbate 20 was then added and stirred in well. The colour was added and the emulsion was then homogenised for a further 5 to 10 minutes until the product was smooth. Purified water, sufficient to make it up to bulk was then added.

### Example 9 - Cleansing Lotion + High Surface Area Zinc Oxide

| Ingredients | % |
|---|---|
| High Surface Area Zinc Oxide | 5.00 |
| Purified Water | to 100 |
| Colour | qs |
| Melaleuca alternifolia | 0.50 |
| Polypropylene glycol-5-ceteth-20 | 3.25 |
| Polysorbate 80 | 0.20 |
| Citric acid | 0.12 |
| Disodium Phosphate | 0.38 |
| Triclosan | 0.30 |
| Butylene Glycol | 0.20 |
| Alcohol (denatured) | 48.00 |

### Stage 1

Alcohol (denatured) and triclosan were mixed together until homogeneous. Water was added and mixed well. Butylene glycol was then added and the mixture stirred.

### Stage 2

To a suitable stainless steel container disodium phosphate and water were added, and warmed to 55-60°C with stirring. Then more water was added, with stirring and the solution was then allowed to cool.

### Stage 3

When the temperature of stage 2 had reached 20-25°C it was added to stage 1 with stirring. Citric acid was added and mixed well. In a suitable stainless steel container some of the polypropylene glycol-5-ceteth-20, the melaleuca alternifolia and high surface area zinc oxide were added and mixed thoroughly and then added to the main vessel. In a suitable container the remaining polypropylene glycol-5-ceteth-20 and polysorbate 80 which was then added to the main vessel. Colour was added, followed by water to make up to bulk.

### Example 10 - Toner and Cleanser + High Surface Area Zinc Oxide

| Ingredients | % |
|---|---|
| Purified water | 43.30 |
| Citric acid | 0.12 |
| Sodium citrate | 0.38 |
| Herbal extract | 1.00 |
| Glycerin | 2.00 |
| Polysorbate 80 | 0.20 |
| Alcohol denatured | 48.00 |
| High surface area zinc oxide | 5.00 |

The ingredients were added together and stirred until fully dissolved.

### Example 11 - Light Moisture Fluid + High Surface Area Zinc Oxide

| Ingredients | % |
|---|---|
| Pentaerythrityl tetraisostearate | 4.00 |
| Xanthan gum | 0.25 |
| Tetrasodium EDTA | 0.10 |
| Glycerin | 1.00 |
| Butylene glycol | 2.00 |
| Methylparaben | 0.20 |
| Polyethylene ester-2-stearyl alcohol | 2.00 |
| Polyethylene ester-21-stearyl alcohol | 1.00 |
| Cetyl alcohol | 2.50 |
| Hydroxyethyl cellulose | 0.40 |
| Cetearyl isononanoate | 2.00 |
| Purified water | 76.84 |
| Butylated hydroxytoluene | 0.02 |
| Butyl methoxydibenzoylmethane | 0.50 |
| Octyl methoxycinnamate | 0.99 |
| 2-Bromo-2-nitropropane-1,3-diol | 0.03 |
| Citric acid | 0.06 |
| Sodium citrate | 0.11 |
| Silk powder | 0.50 |
| Herbal extract | 0.40 |
| Propylparaben | 0.10 |
| High surface area zinc oxide | 5.00 |

Hydroxyethylcellulose was dispersed into an aqueous solution of tetrasodium EDTA. Xanthan gum in glycerin was added to the mixture, followed by methyl paraben. The mixture was stirred and warmed to 70-75°C to create the water phase.

Cetyl alcohol, high surface area zinc oxide, polyethylene ester-2-stearyl alcohol, polyethylene ester-21-stearyl alcohol, propyl paraben, cetearyl isononanoate, pentaerythrityl tetraisostearate, butylated hydroxy toluene, butyl methoxydibenzoyl methane and octyl methoxycinnamate were mixed together and warmed to 70-75°C to create the oil phase.

The oil and water phases were then emulsified together for 5-10 minutes before being force cooled to 30-35°C. An aqueous solution of citric acid, sodium citrate and silk powder was added to the emulsion. Herbal extracts were then added, and the emulsion made up to bulk with water.

### Example 12 - Cleansing Wash + High Surface Area Zinc Oxide

| Ingredients | % |
|---|---|
| Cocamidopropyl betaine 30% | 5.00 |
| Benzophenone - 4 | 0.10 |
| Sodium citrate | 0.60 |
| Disodium undecylenamido MEA-sulfosuccinate solution | 1.00 |
| Triclosan | 0.20 |
| Sodium chloride | 1.00 |
| Laureth - 3 | 2.00 |
| Sodium laureth sulfate | 47.20 |
| Melaleuca alternifolia | 0.500 |
| Phenoxyethanol | 0.15 |
| Colour | qs |
| Citric acid | 0.10 |
| Purified Water | to 100 |
| High Surface Area Zinc Oxide | 5.00 |

### Stage 1

In a vessel sodium laureth sulfate, melaleuca alternifolia and high surface area zinc oxide were mixed until uniform.

### Stage 2

In the base pan the triclosan was dispersed in cocamidopropyl betaine 30% and stirred for 5 minutes. The purified water was added and stirred well. Citric acid was added and stirred until dissolved followed by sodium citrate, which was stirred until dissolved. The mixture was then stirred for a further 10 minutes and subseqently cooled to 30-35°C. Premixed phenoxyethanol in water, followed by benzophenone-4 in water were then added. Colour was added followed by saline solution, then water was added to make up to bulk.

### Example 13 - Moisture Fluid + High Surface Area Zinc Oxide

| Ingredients | % |
|---|---|
| High Surface Area Zinc Oxide | 5.00 |
| Glyceryl stearate | 1.50 |
| Steareth-2 | 2.00 |
| Steareth-21 | 1.00 |
| Cetyl Alcohol | 1.00 |
| Glycerin | 1.00 |
| Butylene Glycol | 2.00 |
| Purified Water | 86.50 |

### Stage 1

Glyceryl stearate, steareth-2, steareth-21, cetyl alcohol and high surface area zinc oxide were melted together at 70-75°C.

### Stage 2

Glycerin was dissolved with stirring in water at 70-75°C.

### Stage 3

Stage 1 was then added to stage 2 with stirring, then homogenised for 15 minutes. Water was added to the stirred mixture, then the mixture was cooled to 35°C. Butylene glycol was added and the mixture stirred until homogeneous, then made up to bulk with water.

### Example 14 - Pressed Powder + High Surface Area Zinc Oxide

| Ingredients | % |
|---|---|
| High Surface Area Zinc Oxide | 5.00 |
| Sanitised talc | 85.72 |
| Magnesium Stearate | 5.00 |
| Methylparaben | 0.10 |
| Red colour | 0.23 |
| Yellow colour | 0.45 |
| Paraffinum liquidum | 2.10 |
| Petrolatum | 1.40 |

Sanitised talc, magnesium stearate, methylparaben, high surface area zinc oxide and colours were mixed together for 10 minutes at high speed. Paraffinum liquidum and petrolatum were mixed together, heated to 75° C and then sprayed into the bulk mixture at low speed. The bulk was mixed for 5 minutes, then passed twice through a hammer mill before being passed through a 30 mesh seive.

### Example 15 - Cover Up Stick + High Surface Area Zinc Oxide

| Ingredient | % |
|---|---|
| Chalk | 23.31 |
| Carnauba | 1.29 |
| Candelilla Cera | 1.01 |
| Hydrocarbon consisting of Cera Microcristallina, paraffin and polyethylene | 4.60 |
| Cera Microcristallina | 4.22 |
| Butylated hydroxyacetone | 0.03 |
| Propylparaben | 0.10 |
| Octyldodecanol | 46.02 |
| Triclosan | 0.19 |
| Allantoin | 0.14 |
| Pigment | 11.98 |
| High Surface Area Zinc Oxide | 5.00 |
| Synthetic wax | 2.01 |

Pigments, high surface area zinc oxide and chalk were added to the Diosna mixer and mixed for 30 minutes. The mix was then passed through the Mikro mill, then a vibrating sieve to give the colour preparation.

White wax, Carnauba and candelilla cera were added to a stainless steel steam jacketed pan fitted with a premier dispersator head, and melted together at 90-95°C. To the melt was added the hydrocarbon wax. When melted, ocyldodecanol was added and the mixture stirred.

The mixture was cooled to 85-90°C then propylparaben, butylated hydroxyacetone and triclosan were added to the stirred mixture, followed by allantoin and then by the colour preparation. The mixture was then stirred for a further ten minutes.

The mixture was then stirred through a 40 mesh sieve into a shallow tray and stirred slowly until set.

### Example 16 - Shower Gel + High Surface Area Zinc Oxide

| Ingredients | % |
|---|---|
| Purified Water | 47.48 |
| Perfume | 0.10 |
| Colour | 0.00075 |
| Sodium chloride | 1.19 |
| Dichlorobenzyl Alcohol | 0.50 |
| Butylated Hydroxytoluene | 0.0048 |
| Triclosan | 0.2916 |
| PEG - 7 Glyceryl Cocoate | 2.916 |
| Citric Acid | 0.0216 |
| | |
| A preservative blend consisting of: Phenoxyethanol, Butylparaben, Ethylparaben, Methylparaben and Propylparaben | 0.80 |
| Cocamidopropyl Betaine | 5.83 |
| Sodium Laureth Sulfate | 45.89 |
| High Surface Area Zinc Oxide | 5.00 |

High surface area zinc oxide was added to purified water and mixed well. Sodium chloride and citric acid were then added and the mixture stirred until both had dissolved. Sodium laureth sulfate, cocamidopropyl betaine, phenoxyethanol, butylparaben, ethylparaben, methylparaben and propylparaben were then added and the mixture stirred.

PEG 7 Glyceryl cocoate, butylated hydroxytoluene, triclosan and dichlorobenzyl alcohol were mixed together and warmed to 45°C. Perfume was then added and the mixture stirred until homogenous.

The two mixtures were then combined and stirred until homogenous. Colour solution was added to the stirred mixture followed by water to make up to bulk. The mixture was then stirred until uniform.

### Example 17 - Acne Lotion + High Surface Area Zinc Oxide

| Ingredients | % |
|---|---|
| Purified Water | 85.115 |
| Benzoyl Peroxide | 6.667 |
| Hydroxyethylcellulose | 1.00 |
| Citric Acid | 1.53 |
| Sodium Hydroxide | 0.6915 |
| High Surface Area Zinc Oxide | 5.00 |

Sodium hydroxide was added to a stirred aqueous solution of citric acid. Hydroxyethylcellulose was then added to the mixture which was then stirred for 30 minutes. Benzoyl peroxide was then added followed by some water. The mix was stirred for 2 minutes then homogenised for 20 minutes under vacuum. High surface area zinc oxide was then added to the mixture and stirred thoroughly. Aqueous sodium hydroxide was then added to the stirred mixture, which was then stirred for a further hour.

### Example 18 - Sensitive Cleansing Pads + High Surface Area Zinc Oxide

| Ingredients | % |
|---|---|
| Purified Water | 68.53 |
| A preservative blend consisting of: Phenoxyethanol | |
| Butylparaben, Ethylparaben, Methylparaben and | |
| Propylparaben | 0.80 |
| Perfume | 0.05 |
| Ethoxylated (20) sorbitan monolaurate | 1.02 |
| Sodium Citrate | 0.21 |
| Citric Acid | 0.06 |
| Cetrimoniumbromide | 0.51 |
| Alcohol (denatured) | 17.38 |
| PPG - 5 - Ceteth - 20 | 0.31 |
| Butylene Glycol | 4.60 |
| Glycerin | 1.02 |
| Chlorhexidine digluconate | 0.51 |
| High Surface Area Zinc Oxide | 5.00 |

Chlorhexidine digluconate, butylene glycol, PPG-5-Ceteth-20, glycerin, cetrimoniumbromide, citric acid, sodium citrate were dissolved in purified water. Denatured alcohol, high surface area zinc oxide and the preservative blend were then added and the mixture stirred. A mixture of ethoxylated (2) sorbitan monolaurate and perfume was then added and the mixture stirred. Purified water was added to make up to bulk. The mixture was stirred for 30 minutes then pumped through an 80 mesh sieve to a suitable storage vessel. Rayon/polyester pads were then impregnated with the mixture to give the sensitive cleaning pads.

### Example 19 - Overnight Gel + High Surface Area Zinc Oxide

| Ingredients | % |
|---|---|
| Purified Water | 50.6 |
| Perfume | 0.05 |
| Triclosan | 0.10 |
| Dichlorobenzyl Alcohol | 0.50 |
| Alcohol (denatured) | 39.50 |
| Hydroxyethylcellulose | 1.25 |
| Glycerin | 3.00 |
| High Surface Area Zinc Oxide | 5.00 |

Hydroxyethylcellulose and glycerin were dispersed in water then transferred to a mixer via a sieve covered with muslim.

Denatured alcohol, dichlorobenzyl alcohol and triclosan were mixed together until homogenous. Perfume and then high surface area zinc oxide were then dispersed in the mixture. The mixture was then transferred to the mixer under vacuum via a sieve. The mixture was stirred for 30 minutes until homogeneous. Water was then added to make up to bulk.

### Example 20 - Emergency Gel + High Surface Area Zinc Oxide

| Ingredients | % |
|---|---|
| Purified Water | 37.00 |
| Hydroxypropyl Methylcellulose | 2.50 |
| Sodium Citrate | 0.30 |
| Alcohol (denatured) | 20.00 |
| Butylene Glycol | 15.00 |
| Propylene Glycol | 18.00 |
| Triclosan | 0.20 |
| Salicylic Acid | 2.00 |
| High Surface Area Zinc Oxide | 5.00 |

Propylene glycol, butylene glycol and ethanol were mixed together. Salicylic acid and triclosan were then dissolved in the mixture. High surface area zinc oxide was then added to the stirred mixture. Hydroxypropyl methylcellulose was then dispersed in the mixture and the mixture was stirred for 30 minutes. Aqueous sodium citrate was then added to the mixture under vacuum and the mixture stirred for a further 30 minutes.

Tests were conducted to measure the ability of high surface area zinc oxide to absorb sebum.

Unless otherwise stated, the zinc oxide used in the following examples has an average surface area of 90m²/g and an average particle diameter of 10.47^{µ}m.

This method was taken from American Society For Testing and Materials, 1962, Standard Method of Test for Oil Absorption of Pigments by Spatula Rub-Out. ASTM Standards 1961, p213-214.

The recipe for the artificial sebum used was from a paper called "An Original Procedure for Quantitation of Cutaneous Reabsorption of Sebum" by D Blane, D Saint-Leger, J Bandt, S Constans and P Agache, Arch Dermatol Res, 1989, 281: p346-350:-

| | |
|---|---|
| Palmitic Acid | 5.000g |
| Squalene | 15.000g |
| Cholesterol | 6.000g |
| Oleic Acid | 25.000g |
| Lauric Acid | 1.000g |
| Dervacid 3155 | 3.000g |
| Myristic Acid | 3.000g |
| Evening Primrose Oil deodorised | 34.000g |
| Glycerol Isostearate | 3.000g |
| Coronet Lanolin | 5.000g |
| Total | 100.000g |

These were added together and then heated to form the artificial sebum.
1g of high surface area zinc oxide powder was weighed onto a piece of glass. A beaker of artificial sebum and a pipette were weighed. Artificial sebum was added to the high surface area zinc oxide drop by drop, and mixed thoroughly after each drop was added. The test was complete when exactly enough sebum was added to the high surface area zinc oxide to produce a very stiff, putty like paste, which did not break or separate. The beaker and pipette were weighted to determine the amount of sebum used. This was then repeated. This was done again using zinc oxide BP and Sanitised talc as controls.

**Table 1**

| Table to Show the Sebum Absorbency of High Surface Area Zinc Oxide Compared to Zinc Oxide BP | |
|---|---|
| High Surface Area Zinc Oxide (g) | ZnO BP (g) |
| Amount of Sebum Absorbed | Amount of Sebum Absorbed |
| 1.41 | 0.57 |
| 1.10 | 0.60 |
| 1.25 | 0.45 |
| 1.24 | 0.53 |
| 1.25 | 0.67 |
| 1.22 | 0.64 |
| 1.67 | 0.37 |
| 1.22 | 0.57 |
| 1.45 | 0.56 |
| 1.32 | 0.56 |
| Averages 1.31 | Averages 0.55 |

| | |
|---|---|
| Expressed as a % difference *, these results show that high surface area zinc oxide absorbs an average 76% more artificial sebum than zinc oxide BP * % difference is the difference between the amount of sebum absorbed by high surface area zinc oxide and the amount absorbed by zinc oxide BP, relative to the amount of sebum absorbed by zinc oxide BP. | |

**Table 2**

| Table to Show the Sebum Absorbency of High Surface Area Zinc Oxide Compared to Sanitised Talc | |
|---|---|
| High Surface Area Zinc Oxide (g) | Sanitised Talc |
| Amount of Sebum Absorbed | Amount of Sebum Absorbed |
| 1.41 | 0.84 |
| 1.10 | 0.81 |
| 1.25 | 0.72 |
| 1.24 | 0.76 |
| 1.25 | 0.92 |
| 1.22 | 0.87 |
| 1.67 | 0.64 |
| 1.22 | 1.59 |
| 1.45 | 0.85 |
| 1.32 | 0.94 |
| Averages | Averages |
| 1.31 | 0.89 |

| | |
|---|---|
| Expressed as a % difference *, these results show that high surface area zinc oxide absorbs on average 47% more artificial sebum than sanitised talc. * % difference is the difference between the amount of sebum absorbed by high surface area zinc oxide and the amount absorbed by talc, relative to the amount of sebum absorbed by talc. | |

Overall the high surface area zinc oxide is better at absorbing artificial sebum than Zinc Oxide BP and Sanitised Talc.

### Sebum Fatty Acid Absorption

Sebum fatty acid absorption by high surface area zinc oxide was analysed using gas chromatography.

### Method

The artificial sebum was prepared for analysis in the following way: 1% of high surface area zinc oxide was put into a beaker with 99% of artificial sebum. This was mixed for half an hour with a magnetic stirrer, to make sure that the high surface area zinc oxide was fully mixed in and in contact with all of the sebum. The solution was then centrifuged for two minutes at 14,000 rpm. The supernatant was removed and samples of this were sent for fatty acids analysis using gas chromatography. This was repeated for a mixture containing 1.33% high surface area zinc oxide and 98.66% artificial sebum. A sample of artificial sebum which had not had any high surface area zinc oxide added to it was used as a control.

**Table 3**

| Table to Show the Absorption of Specific Fatty Acids by the High Surface Area Zinc Oxide | | | | | | |
|---|---|---|---|---|---|---|
| Fatty Acid | Chain Length | Sebum only (control) | Sebum With 1% ZnO S | Percentage Fatty Acid Absorbed | Sebum with 1.33% ZnO S | Percentage Fatty Acid Absorbed |
| % Lauric Acid | C12 | 0.95 | 0.90 | 5.26 | 0.93 | 2.11 |
| % Myristic Acid | C14 | 3.17 | 3.11 | 1.89 | 2.81 | 11.36 |
| % Palmitic Acid | C16 | 6.17 | 5.20 | 15.72 | 5.10 | 17.34 |
| % Stearic Acid | C18 | 3.67 | 3.07 | 16.35 | 3.08 | 16.08 |
| % Oleic Acid | C18, with 1 unsaturated bond | 28.60 | 25.10 | 12.24 | 25.30 | 11.54 |

These results show that high surface area zinc oxide preferentially absorbs these longer chain fatty acids. Longer chain fatty acids are more comedogenic than shorter chain fatty acids.

Tests were conducted to measure the ability of high surface area zinc oxide to absorb sweat, in a similar to that described above for sebum absorption.

### Method

This method was take from American Society for Testing and Materials. 1962. Standard Method of Test for Oil Absorption of Pigments by Spatula Rub-Out. ASTM Standards 1961, p213-214 and modified for measuring sweat absorption.

The recipe for the artificial sweat was taken and modified from Geigy Scientific Tablets. Units of Measurements. Body Fluids. Composition of The Body. Nutrition. C Lentner. Ciba-Geigy. 1981. p108-112:

| | |
|---|---|
| Water | 994.060g |
| Urea | 1.990g |
| Glucose | 0.170g |
| Lactic Acid | 1.190g |
| Leucine | 2.590g |
| Total | 1000.000g |

The above raw materials were dissolved together to form the artificial sweat.

**Table 4**

| Table to Show the Sweat Absorbency of High Surface Area Zinc Oxide Compared to Zinc Oxide BP | |
|---|---|
| High Surface Area ZnO (g) | ZnO BP (g) |
| Amount of Sweat Absorbed | Amount of Sweat Absorbed |
| 2.90 | 0.60 |
| 1.40 | 0.50 |
| 2.00 | 0.60 |
| 1.60 | 0.60 |
| 1.40 | 0.20 |
| 1.60 | 0.50 |
| 1.30 | 1.10 |
| 1.50 | 0.70 |
| 1.20 | 0.50 |
| 1.50 | 0.50 |
| Averages | Averages |
| 1.64 | 0.58 |

| | |
|---|---|
| Expressed as a % difference *, these results show that high surface area zinc oxide absorbs on average 183% more artificial sweat than zinc oxide BP Thus these results show that high surface area zinc oxide absorbs artificial sweat better than Zinc Oxide BP. * % difference is the difference between the amount of sweat absorbed by high surface area zinc oxide and the amount absorbed by zinc oxide BP, relative to the amount of sweat absorbed by zinc oxide BP. | |

**Table 5**

| Table to Show the Sweat Absorbency of High Surface Area Zinc Oxide Compared to Sanitised Talc | |
|---|---|
| High Surface Area ZnO (g) | Sanitised Talc (g) |
| Amount of Sweat Absorbed | Amount of Sweat Absorbed |
| 2.90 | 1.00 |
| 1.40 | 1.70 |
| 2.00 | 1.10 |
| 1.60 | 0.70 |
| 1.40 | 0.90 |
| 1.60 | 0.90 |
| 1.30 | 0.90 |
| 1.50 | 0.90 |
| 1.20 | 1.10 |
| 1.50 | 0.80 |
| Averages | Averages |
| 1.64 | 1.0 |

| | |
|---|---|
| Expressed as a % difference *, these results show that on average, high surface area zince oxide absorbs 64% more artificial sweat than sanitised talc. Thus these results show that high surface area zinc oxide absorbs artificial sweat better than sanitised talc. * % difference is the difference between the amount of sweat absorbed by high surface area zinc oxide and the amount absorbed by talc, relative to the amount of sweat absorbed by the talc. | |

### Tests were conducted to measure the ability of foot powder formulations to absorb water

1g of foot powder containing high surface area zinc oxide powder (as made in Example 3) was weighed onto a piece of glass. Water was added to the foot powder drop by drop, and mixed thoroughly after each drop was added. The test was completed when exactly enough water was added to the foot powder to produce a very stiff, putty like paste, which did not break or separate. The beaker and pipette were weighed to determine the amount of water used. This was then repeated using a control of a standard foot powder (as made in Example 2).

**TABLE 6**

| Table to show the absorbency of foot powder with high surface area zinc oxide compared to standard without high surface area zinc oxide | |
|---|---|
| Water absorbed by foot powder containing high surface area zinc oxide (g) | Water absorbed by standard foot powder (g) |
| 1.17 | 1.13 |
| 1.26 | 0.94 |
| 1 | 0.97 |
| 1.11 | 0.96 |
| 1.2 | 0.97 |
| 1.15 | 1.13 |
| 1.1 | 1.02 |
| 1.21 | 0.97 |
| 1.05 | 0.98 |
| 1.12 | 0.87 |
| Average | Average |
| 1.37 | 0.994 |

| | |
|---|---|
| Expressed as a % difference *, these results show that on average, foot powder containing high surface area zinc oxide absorbs 15% more water than the standard foot powder. Thus these results show that foot powder containing high surface area zinc oxide is more efficacious at absorbing water than the conventional foot powder formulation. * % difference is the difference between the amount of water absorbed by foot powder containing high surface area zinc oxide, and the amount absorbed by standard foot powder, relative to the amount absorbed by standard foot powder. | |

## Claims

1. The use of high surface area zinc oxide particles having a surface area between 30m²/g and 100m²/g and a particle size between 0.1 and 200µm in diameter and a cosmetically acceptable diluent or carrier, for the preparation of a topical sebum absorbing composition in which said high surface area zinc oxide particles are present in an amount from 1-10% by weight of the total composition and absorb straight chain fatty acids found in sebum from the parts of the body to which the topical composition is applied.

2. A use as claimed in claim 1 where the surface area of the high surface area zinc oxide is between 90 and 100m²/g and the particle size is between 0.1 and 20.5µm in diameter.

3. A use as claimed in claim 1 where the high surface area zinc oxide is present from 3 to 8% by weight of the total composition.

4. A use as claimed in claim 1 where the high surface area zinc oxide is present from 4 to 6% by weight of the total composition.

5. A use as claimed in any one of the preceding claims wherein the composition is a medicament for the treatment of greasy skin, greasy hair, acne, athletes foot and nappy rash.

6. A use as claimed in claim 1 where the high surface area zinc oxide absorbs one or more of the fatty acids selected from lauric acid, myristic acid, palmitic acid, stearic acid and oleic acid.

7. A use as claimed in Claim 1 where the high surface area zinc oxide absorbs stearic acid and palmitic acid in preference to shorter chain fatty acids found in sebum.

## Patentansprüche

1. Verwendung von Zinkoxidteilchen mit hoher Oberfläche mit einer Oberfläche zwischen 30 m²/g und 100 m²/g und einer Teilchengröße zwischen 0,1 und 200 µm im Durchmesser und einem kosmetisch verträglichen Verdünnungsmittel oder einem kosmetisch verträglichen Träger, zur Herstellung einer topischen, Sebum absorbierenden Zusammensetzung, wobei die Zinkoxidteilchen mit hoher Oberfläche in einer Menge von 1 - 10 Gew.% der Gesamtzusammensetzung vorliegen und geradkettige Fettsäuren, die im Sebum von Teilen des Körpers vorkommen, auf die die topische Zusammensetzung aufgetragen wird, absorbieren.

2. Verwendung nach Anspruch 1, wobei die Oberfläche von dem Zinkoxid mit hoher Oberfläche zwischen 90 und 100 m²/g liegt und die Teilchengröße zwischen 0,1 und 20,5 µm im Durchmesser ist.

3. Verwendung nach Anspruch 1, wobei das Zinkoxid mit hoher Oberfläche von 3 bis 8 Gew.-% der Gesamtzusammensetzung vorliegt.

4. Verwendung nach Anspruch 1, wobei das Zinkoxid mit hoher Oberfläche von 4 bis 6 Gew.-% der Gesamtzusammensetzung vorliegt.

5. Verwendung nach einem der vorangehenden Ansprüche, wobei die Zusammensetzung ein Medikament für die Behandlung von fettiger Haut, fettigem Haar, Akne, Fußpilz und Windelekzem ist.

6. Verwendung nach Anspruch 1, wobei das Zinkoxid mit hoher Oberfläche eine oder mehrere Fettsäuren, ausgewählt aus Laurinsäure, Myristinsäure, Palmitinsäure, Stearinsäure und Olsäure, absorbiert.

7. Verwendung nach Anspruch 1, wobei das Zinkoxid mit hoher Oberfläche Stearinsäure und Palmitinsäure in Bevorzugung zu kürzerkettigen Fettsäuren, die im Sebum vorkommen, absorbiert.

## Revendications

1. Utilisation de particules d'oxyde de zinc de surface spécifique élevée ayant une surface spécifique comprise entre 30 m²/g et 100 m²/g et une taille des particules comprise entre 0,1 et 200 µm en termes de diamètre et d'un diluant ou véhicule acceptable du point de vue cosmétique, pour la préparation d'une composition absorbant le sébum à usage local dans laquelle lesdites particules d'oxyde de zinc de surface spécifique élevée sont présentes en quantité allant de 1 à 10 % en poids de la composition totale et absorbent les acides gras à chaîne linéaire trouvés dans le sébum des parties du corps sur lesquelles la composition à usage local est appliquée.

2. Utilisation selon la revendication 1 dans laquelle la surface spécifique de l'oxyde de zinc de surface spécifique élevée est comprise entre 90 et 100 m²/g et la taille des particules est comprise entre 0,1 et 20,5 µm en termes de diamètre.

3. Utilisation selon la revendication 1 dans laquelle l'oxyde de zinc de surface spécifique élevée est présent en quantité allant de 3 à 8 % en poids de la composition totale.

4. Utilisation selon la revendication 1 dans laquelle l'oxyde de zinc de surface spécifique élevée est présent en quantité allant de 4 à 6 % en poids de la composition totale.

5. Utilisation selon l'une quelconque des revendications précédentes dans laquelle la composition est un médicament pour le traitement des peaux grasses, des cheveux gras, de l'acné, du pied d'athlète et de l'érythème fessier du nourrisson.

6. Utilisation selon la revendication 1 dans laquelle l'oxyde de zinc de surface spécifique élevée absorbe un ou plusieurs des acides gras choisis entre l'acide laurique, l'acide myristique, l'acide palmitique, l'acide stéarique et l'acide oléique.

7. Utilisation selon la revendication 1 dans laquelle l'oxyde de zinc de surface spécifique élevée absorbe l'acide stéarique et l'acide palmitique de préférence à des acides gras à plus courte chaîne trouvés dans le sébum.
